# EUROPEAN PATENT SPECIFICATION

(11) **EP 3 631 470 B1**
(45) Date of publication and mention of the grant of the patent: **31.12.2025**
(21) Application number: 18806705.2
(22) Date of filing: 25.05.2018
(51) Int. Cl.: G01N 33/68, A61P 25/00

(54) **DETERMINING ONSET OF AMYOTROPHIC LATERAL SCLEROSIS**
BESTIMMUNG DES EINSETZENS VON AMYOTROPHER LATERALSKLEROSE
DÉTERMINATION DE L'APPARITION DE LA SCLÉROSE LATÉRALE AMYOTROPHIQUE

(30) Priority: 26.05.2017 US 201762511595 P
(43) Date of publication of application: 08.04.2020
(73) Proprietor: University of Miami, Miami, FL 33136 (US); Queen Mary University of London, London E1 4NS (GB)
(72) Inventor: BENATAR, Michael, Miami, FL 33129 (US); MALASPINA, Andrea, London E1 4NS (GB)
(74) Representative: Inspicos P/S
(86) International application number: PCT/US2018/034765
(87) International publication number: WO 2018/218219

(56) References cited:
- WO-A1-2015/157300
- WO-A2-2015/153800
- US-A1- 2009 053 723
- US-A1- 2013 280 733
- US-A1- 2015 285 822
- US-A1- 2016 091 504
- US-A1- 2016 120 831
- WEYDT P. ET AL.: "Neurofilament levels as biomarkers in asymptomatic and symptomatic familial amyotrophic lateral sclerosis", ANN. NEUROL., vol. 79, 2016, pages 152 - 158, XP055772130
- BENATAR M. ET AL.: "Neurofilament light: A candidate biomarker of presymptomatic amyotrophic lateral sclerosis and phenoconversion", ANN. NEUROL., vol. 84, July 2018 (2018-07-01), pages 130 - 139, XP055669467
- BENATAR M. ET AL.: "Neurofilaments in pre-symptomatic ALS and the impact of genotype", AMYOTROPH. LATERAL SCLER. FRONTOTEMPORAL DEGENER., vol. 20, no. 7-8, 21 August 2019 (2019-08-21), pages 538 - 548, XP055770423
- BJORNEVIK K. ET AL.: "Prediagnostic neurofilament light chain levels in amyotrophic lateral sclerosis", NEUROLOGY, vol. 97, no. 15, 12 October 2021 (2021-10-12), pages e1466 - e1474, XP093257825
- GLOBE NEWSWIRE: "Voyager Therapeutics Highlights Programs in Parkinson’s Disease and ALS; Announces Expansion of CNS Pipeline at R&D Day", CNBC, 29 April 2016 (2016-04-29), XP055552693, Retrieved from the Internet <URL:https://www.cnbc.com/2016/04/29/globe-newswire-voyager-therapeutics-highlights-programs-in-parkinsonas-disease-and-als-announces-expansion-of-cns-pipeline-at-rd-day.html> [retrieved on 20180806]

## Description

### FIELD OF THE INVENTION

The present disclosure relates, in general, to a method of predicting the onset of amyotrophic lateral sclerosis (ALS) in a pre-symptomatic human subject and preventing, delaying the onset of or treating ALS.

### BACKGROUND

Amyotrophic lateral sclerosis (ALS), also known as "Lou Gehrig's disease," is a late-onset fatal neurodegenerative disease characterized by progressive loss of the upper and lower motor neurons at the spinal or bulbar level (Rowland et al., N Engl J Med. 2001;344:1688-700). Approximately 30,000 individuals in the United States have ALS, which can lead to paralysis and also death. Early symptoms of ALS include increasing muscle weakness, especially involving the arms and legs, speech, swallowing and breathing. ALS is categorized in two forms. Most commonly (~90%), ALS occurs sporadically, with an identifiable genetic cause of disease rarely (~5-7%) identified. Less commonly (~10%), ALS runs in families, with a genetic cause of disease identifiable in most patients. In the non-genetic forms of ALS, the etiology of disease remains unknown (Zarei et al., Surg Neurol Int. 2015;6: 171). Weydt et al. states that it is well-known that neurofilaments are elevated in the cerebrospinal fluid (CSF) and serum of amyotrophic lateral sclerosis (ALS) patients, but that timing of this increase is unknown. Thus, to characterize the pre-manifest disease phase, a cross-sectional study on asymptomatic and symptomatic ALS mutation carriers and family controls was performed, that shows that neurofilaments NF-L (neurofilament-light chain) and pNF-H (phosphorylated neurofilament-heavy chain) are normal before symptom onset and increased by at least an order of magnitude at early symptom onset in CSF (pNF-H) or serum and CSF (NF-L). The authors conclude that blood and CSF elevated neurofilament levels are restricted to the symptomatic phase of ALS, and likely are indicators of an ongoing neurodegenerative process (Weydt et al., Ann. Neurol. 2016;79:152-158).

### SUMMARY OF THE INVENTION

The disclosure provides a method of predicting the onset of clinical manifestations of ALS in a pre-symptomatic human subject. An exemplary method comprises the following steps: (a) measuring neurofilament light chain (NfL) and/or phosphorylated neurofilament heavy chain (pNfH) levels in a subject; and (b) predicting the onset of ALS. Increased levels of NfL and/or pNfH signal the onset of clinical manifestations of ALS.

In related embodiments, the subject is a pre-symptomatic human ALS-associated gene mutation carrier. The pre-symptomatic human ALS-associated gene mutation carrier optionally comprises a mutation in *superoxide dismutase 1* (SOD1), *chromosome 9 open reading frame 72* (C9orf72), *fused in sarcoma* (FUS), *TAR DNA Binding Protein* (TARDBP), *valosin-containing protein* (VCP), *Angiogenin* (ANG), *Coiled-coil-helix-coiled-coil-helixdomain containing 10* (CHCHD10), *Chromatin modifying protein 2B* (CHMP2B), *Heterogenous nuclear ribonucleoprotein A*1(HNRNPA1), *Matrin 3* (MATR3), *Optineurin* (OPTN), *Profilin 1* (PFN1), *Spatacsin* (SPG11), *Sequestosome 1* (SQSTM1), *TDP-43* (TARDP), *TANK-binding kinase 1* (TBK1), *Tubulin Alpha 1* (TUBA4A), *Ubiquilin-2* (UBQLN2), or a combination thereof.

In related embodiments, step (a) comprises measuring the level of pNfH and/or NfL protein from blood serum, plasma, and/or cerebrospinal fluid (CSF) of the human subject. In various embodiments, the pNfH and NfL protein levels are measured by antibody-based immunoassays. Alternatively or in addition, step (a) comprises measuring the levels of neurofilament-containing hetero-aggregates, or cleavage products of NfH and/or NfL (or a combination of any of the foregoing).

In various embodiments, the method further comprises (c) administering an ALS therapy to the subject. In related embodiments, ALS therapy is performed within 12-16 months (e.g., 12 months) of step (b). The ALS therapy is optionally selected from the group consisting of edaravone (Radicut^{®} or Radicava^{®}), riluzole (Rilutek^{®}, Teglutik^{®}), mesenchymal stem cells, copper-ATSM (CuATSM), AB-1010 (masitinib), CK-2017357 (tirasemtiv), E0302 (mecobalamin), NP001, pyrimethamine, ibudilast, glial restricted progenitor cells, mexiletine, memantine, GDC-0134, EPI-589, RNS-60, pimozide, tocilizumab, ezogabine, ODM-109, low-dose IL-2, amylyx, an antisense oligonucleotide, gene therapy, and combinations thereof. In related embodiments, the antisense oligonucleotide or gene therapy (e.g., viral-vector mediated gene therapy) targets one or more genetic mutation(s) associated with ALS. In related embodiments, the antisense oligonucleotide is ISIS-SOD1Rx (BIIB067), C9orf72, ASO-816, ASO061, ISIS SMNRx or ISIS 333611. In related embodiments, the gene therapy is Voyager-superoxide dismutase 101 (VY-SOD101).

The disclosure also provides a method comprising (a) measuring phosphorylated neurofilament heavy chain (pNfH) and/or neurofilament light chain (NfL) levels in a pre-symptomatic human ALS-associated gene mutation carrier at two or more time points; and (b) administering an ALS therapy to the subject when (i) absolute NfL levels are at least 24 pg/ml and/or (ii) pNfH and/or NfL levels increase between the measurements taken at two or more time points in step (a).

It is understood that each feature or embodiment, or combination, described herein is a non-limiting, illustrative example of any of the aspects of the disclosure and, as such, is meant to be combinable with any other feature or embodiment, or combination, described herein. For example, where features are described with language such as "one embodiment," "some embodiments," "various embodiments," "related embodiments," each of these types of embodiments is a non-limiting example of a feature that is intended to be combined with any other feature, or combination of features, described herein without having to list every possible combination. Such features or combinations of features apply to any of the aspects of the invention. The invention is defined by the appended claims.

The headings herein are for the convenience of the reader and not intended to be limiting. Additional aspects, embodiments, and variations of the invention will be apparent from the Detailed Description and/or drawings and/or claims.

### BRIEF DESCRIPTION OF THE DRAWINGS

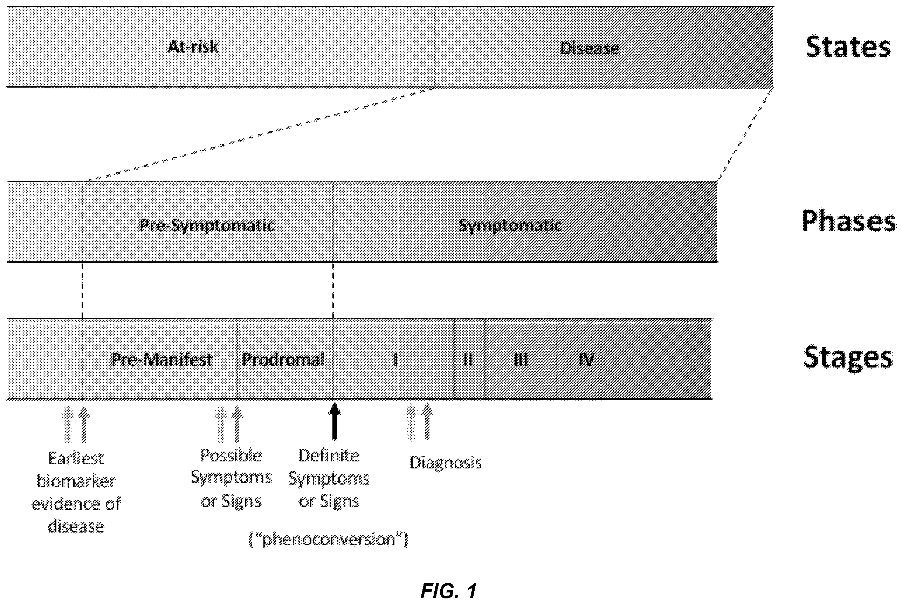

**Figure 1****: Illustration of the progression of ALS.** The progression of ALS, including transitions from the at-risk state to the disease state; from the pre-symptomatic to the symptomatic phase of disease; and from the pre-manifest stage to the prodromal stage of pre-symptomatic disease, are illustrated using a grayscale gradient to reflect the likelihood that these states, phases and stages exist along a continuum. (The figure is not drawn to scale as the relative duration of each state, phase, and stage may vary between individuals).

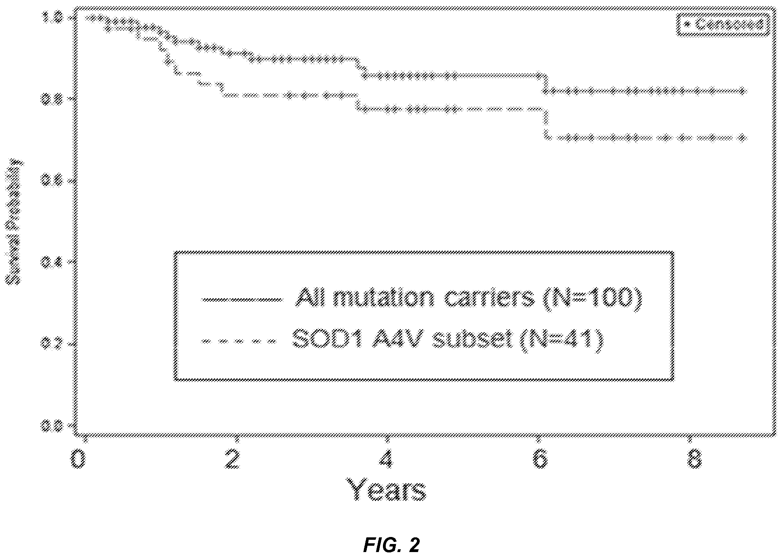

**Figure 2****: Kaplan-Meier estimate of phenoconversion rates in the Pre-Symptomatic Familial ALS (Pre-fALS) Cohort.** 347 person-years follow-up among the 100 gene mutation carriers enrolled in the Pre-Symptomatic Familial ALS Cohort. Figure 2 is a graph comparing survival probability (y-axis) and years (x-axis), where 'survival' reflects freedom from having developed symptomatic or clinically manifest disease. Figure 2 shows the survival probability of all mutation carriers (N=100) and subset of subjects carrying the SOD1 A4V mutation (N=41).

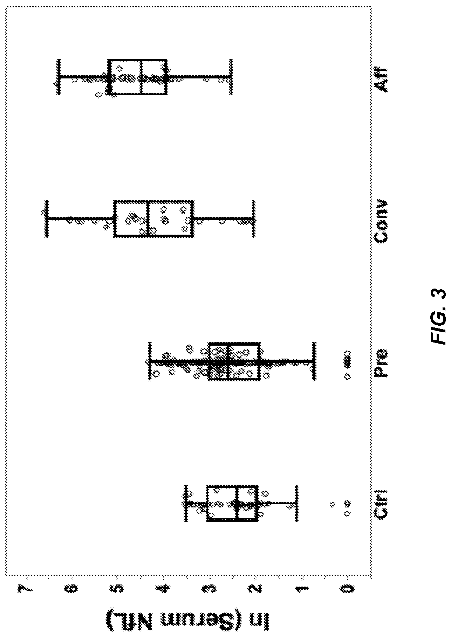

**Figure 3****: Serum neurofilament light (NfL) levels (y-axis) among phenoconverters, ALS patients, compared to healthy controls and pre-symptomatic individuals who have not yet phenoconverted.** NfL was quantified in 34 controls (56 samples), 84 presymptomatic individuals (214 samples), 11 phenoconverters (30 samples) and 19 ALS affected individuals (45 samples). The phenoconverter (Conv) group includes samples collected during the pre-symptomatic and early manifest stages. Figure 3 is a graph comparing the levels of NfL is serum (y-axis) of healthy controls (Crtl), pre-symptomatic individuals who have not yet phenoconverted (Pre), phenoconverters (Conv) and ALS affected individuals (Aff), on the x-axis.

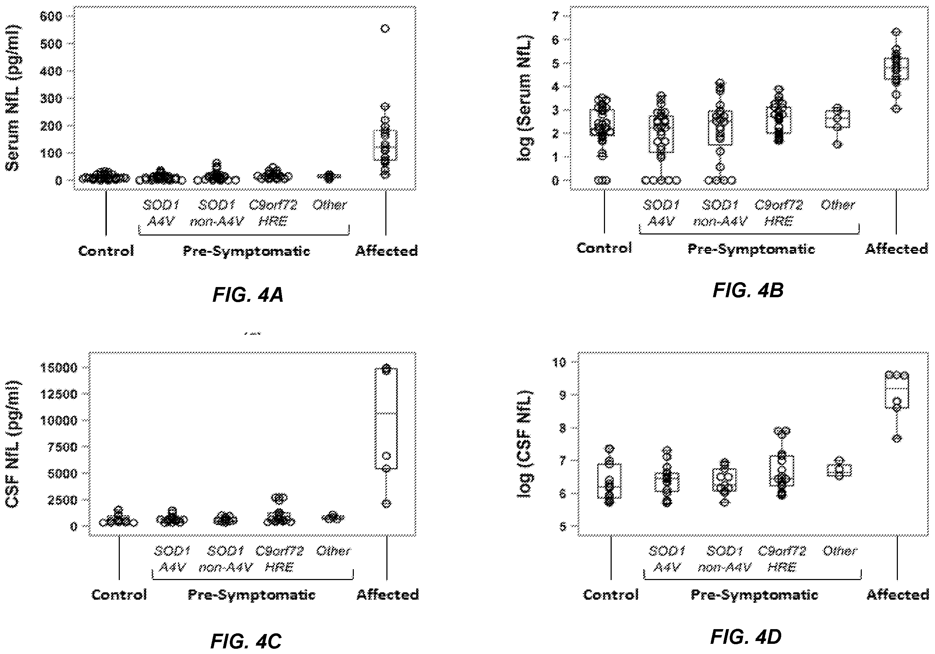

**Figures 4A-4D****: Baseline levels of NfL in serum and CSF: serum NfL (pg/ml) (****Figure 4A****); natural log-transformed serum NfL (****Figure 4B****); CSF NfL (pg/ml) (****Figure 4C****); and natural log-transformed CSF NfL (****Figure 4D****).** Boxes show median, and 25th and 75th percentiles; whiskers extend to a maximum of 1.5 x interquartile range (IQR), or to the most extreme value if it is less than 1.5 x IQR from the 25th or 75th percentile.

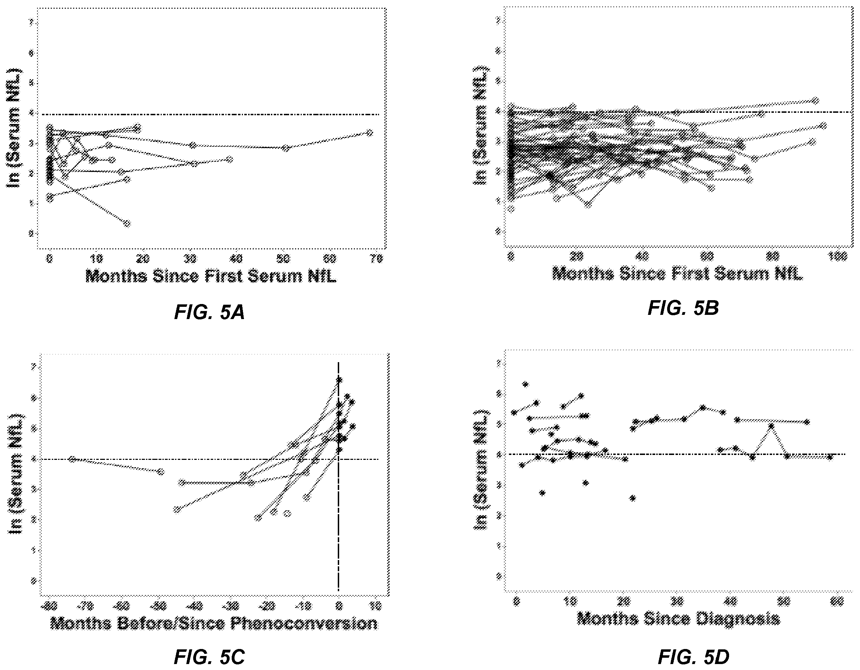

**Figures 5A-5D****: Longitudinal trajectories of serum neurofilament light (NfL) levels in individual subjects.** Levels of NfL in the serum of healthy controls (**Figure 5A**) and pre-symptomatic gene mutation carriers (**Figure 5B**) are shown on a natural logarithmic scale (shown as a faint horizontal dotted line) and largely stable over time. Serum levels of NfL are shown in the months before/since phenoconversion (**Figure 5C**). Serum levels of NfL are shown in the months since diagnosis (**Figure 5D**). Open circles represent samples from individuals at a time when the neurological examination is normal, whereas closed circles represent samples from individuals in whom physical signs of disease are apparent on clinical examination.

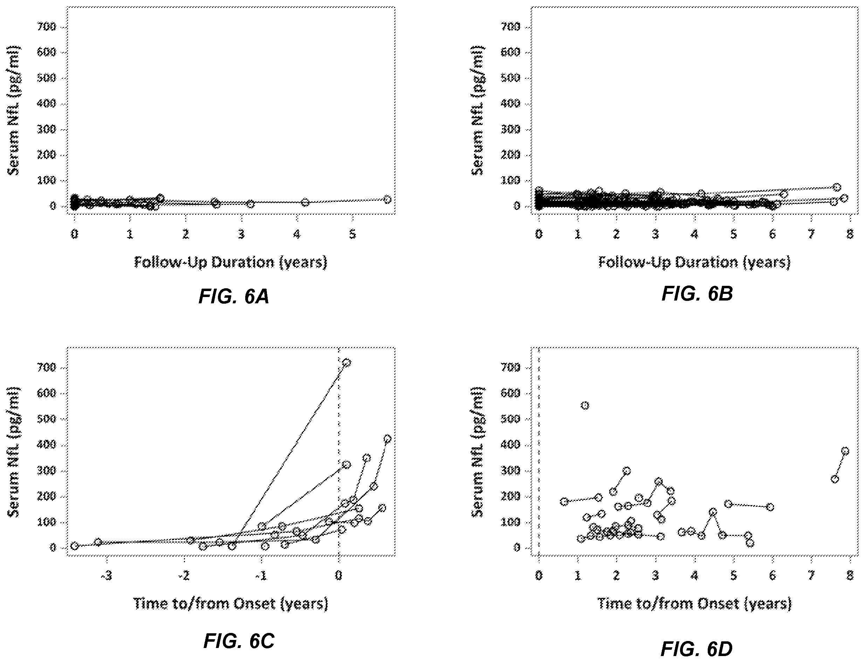

**Figures 6A-6B****: Longitudinal changes in serum NfL concentration (pg/ml)** (Figure 6A, controls; Figure 6B; at-risk individuals who remain pre-symptomatic throughout follow-up; Figure 6C, phenoconverters; and Figure 6D, ALS patients). The x-axis in (a) and (b) shows years since baseline. The x-axis in (c) and (d) shows years to or since the onset of symptoms or signs, which is marked by the vertical dashed line at year=0.

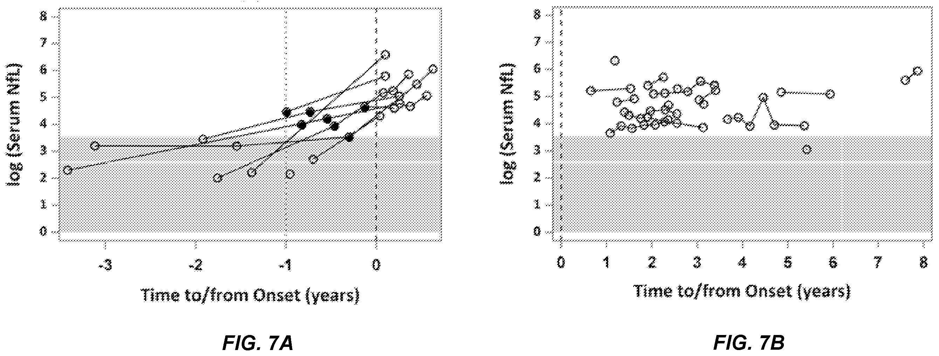

**Figures 7A-7B****: Longitudinal changes in serum NfL concentration, natural log-transformed phenoconverters** (Figure 7A, phenoconverters; Figure 7B, ALS patients). The x-axis shows years to or since the onset of symptoms or signs, which is marked by the vertical dashed line at year=0. The gray area covers the range of serum NfL values observed in the control group. The closed circles mark the elevated levels of NfL (i.e., above the highest value seen in controls) that were measured in serum collected before the onset of symptoms or signs.

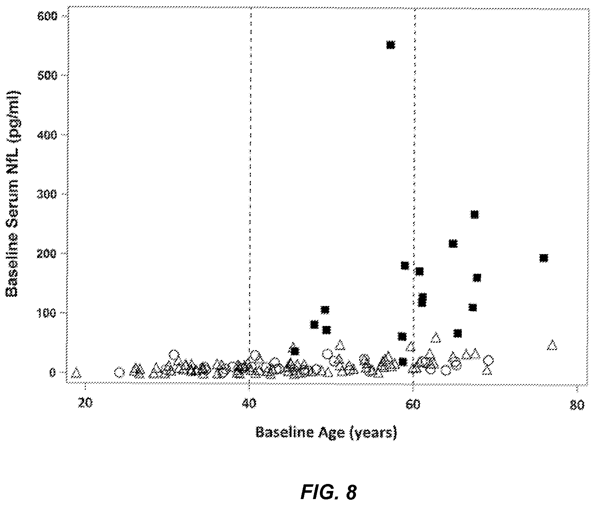

**Figure 8****: Scatterplot of baseline serum NfL levels versus baseline age.** The Figure shows that, although NfL levels are slightly higher among older individuals in the control (open circles) and at-risk groups (open triangles), the magnitude of this increase is negligible in comparison to the levels of NfL observed among ALS (closed squares) patients (i.e., older age does not explain the increase in serum NfL among ALS patients). Open circles represent controls; open triangles represent at-risk individuals who remain pre-symptomatic throughout follow-up; and filled squares represent ALS patients. Vertical dashed lines demarcate age groups (< 40, 40-60 and > 60 years of age).

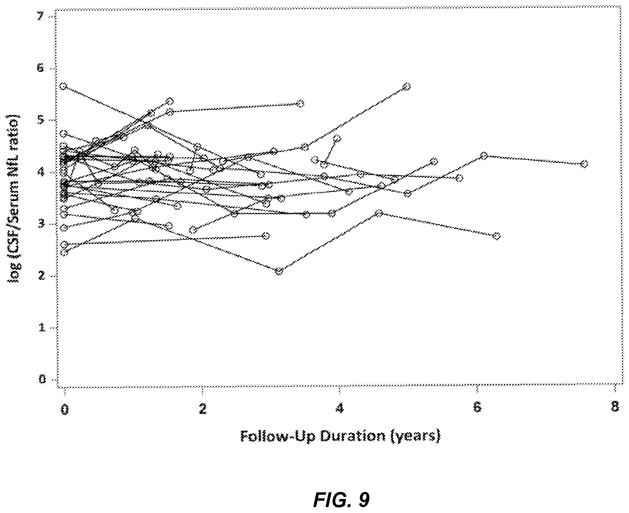

**Figure 9****: The longitudinal trajectory of the CSF/serum ratio of NfL among all study participants (controls, at-risk, phenoconverters and ALS affected) is largely stable over time.** The y-axis shows the natural log of the CSF/serum ratio, and the x-axis shows years of follow-up since baseline.

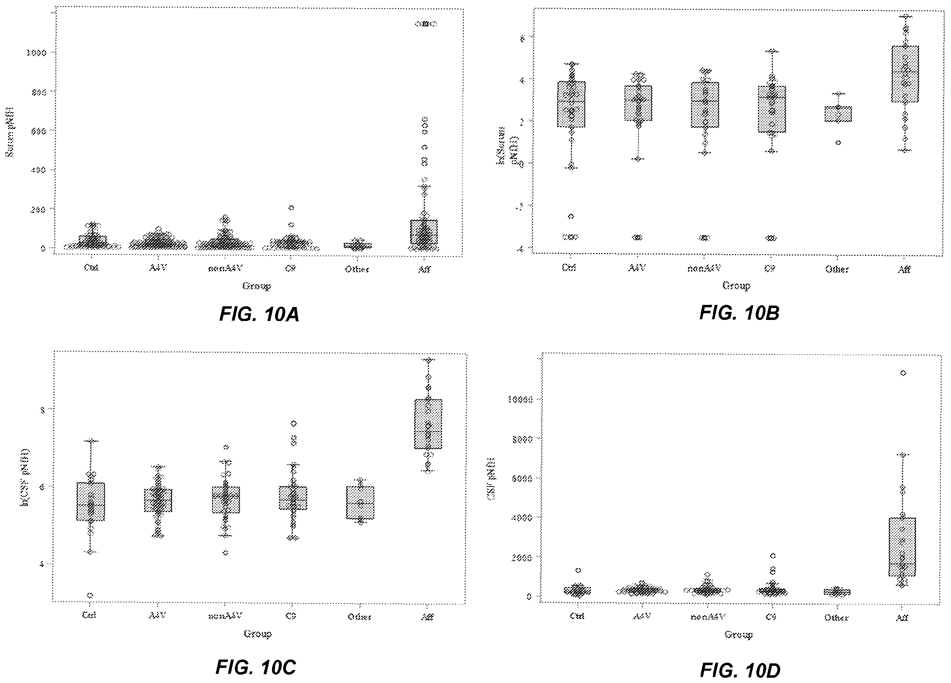

**Figures 10A-10D****: Serum neurofilament heavy (pNfH) levels (y-axis) among phenoconverters, ALS patients, compared to healthy controls and pre-symptomatic individuals who have not yet phenoconverted** (all person-visits). Serum pNfH on the natural scale (Figure 10A). Natural log of serum pNfH (Figure 10B). CSF pNfH on the natural scale (Figure 10C). Natural log of CSF pNfH (Figure 10D).

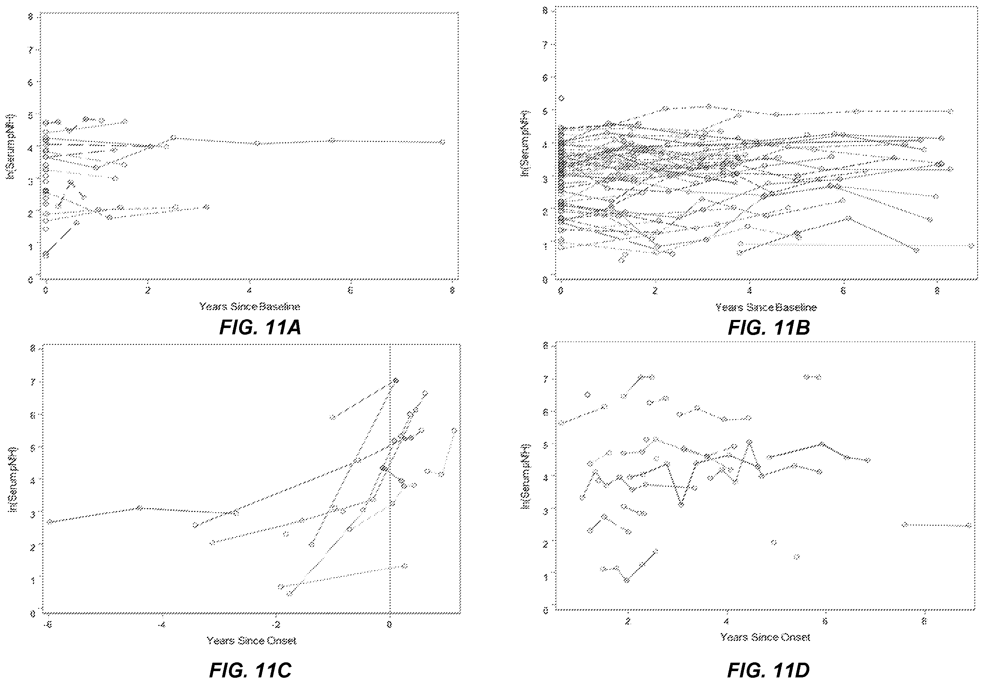

**Figures 11A-11D****: Longitudinal changes in serum pNfH concentration (pg/ml).** Longitudinal trajectory of natural log serum pNfH levels among controls (Figure 11A). Longitudinal trajectory of natural log serum pNfH levels among pre-symptomatic individuals who have not undergone phenoconversion (Figure 11B). Longitudinal trajectory of natural log serum pNfH among phenoconverters (Figure 11C). Longitudinal trajectory of natural log serum pNfH among ALS affected individuals (Figure 11D).

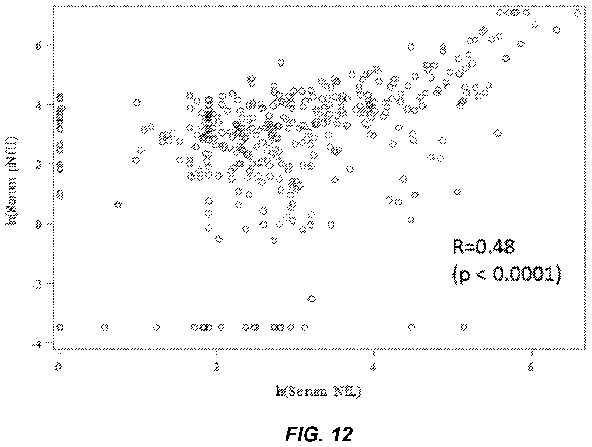

**Figure 12****: Correlation between natural log of serum pNfH and serum NfL. R=0.48 (p < 0.0001).**

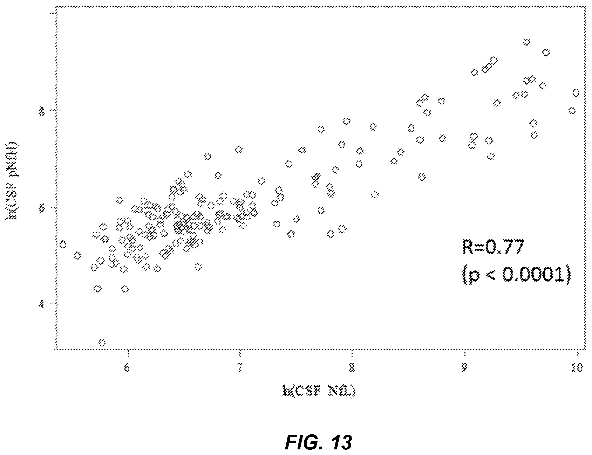

**Figure 13****: Correlation between natural log of CSF pNfH and CSF NfL. R=0.77 (p < 0.0001).**

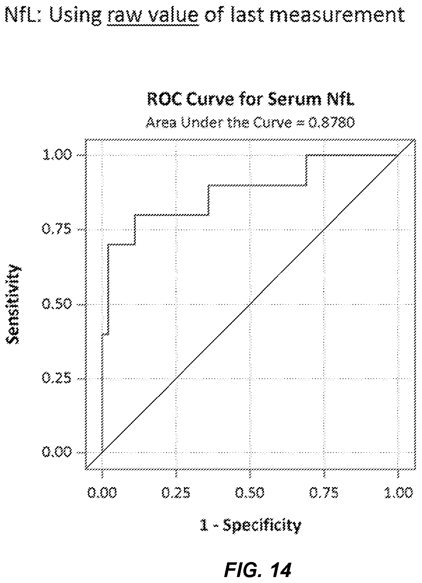

**Figure 14****: Receiver operating characteristic (ROC) analysis of the predictive utility of absolute serum NfL value.** The absolute raw value of serum NfL was used to predict the likelihood of phenoconversion (development of clinically manifest disease) within the following year (Area Under the Curve [AUC] = 0.8780).

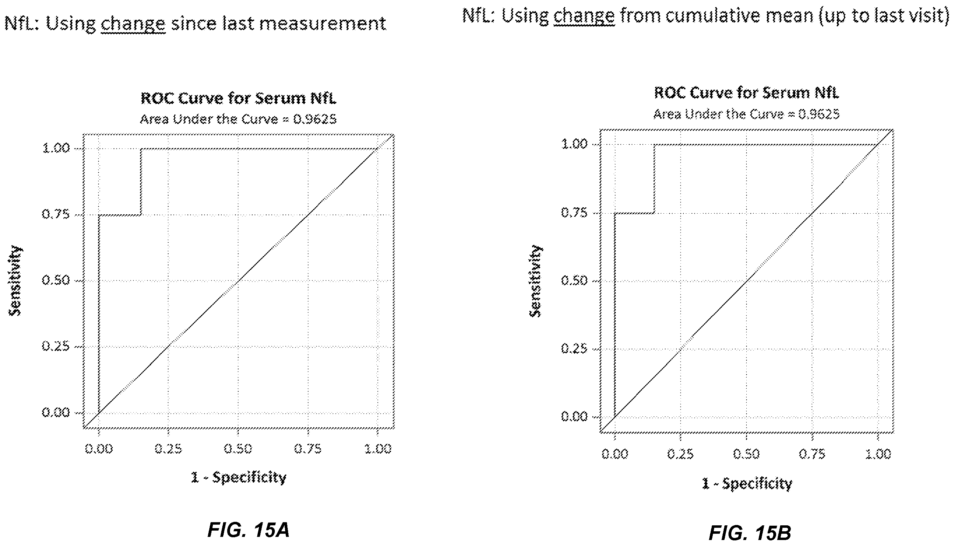

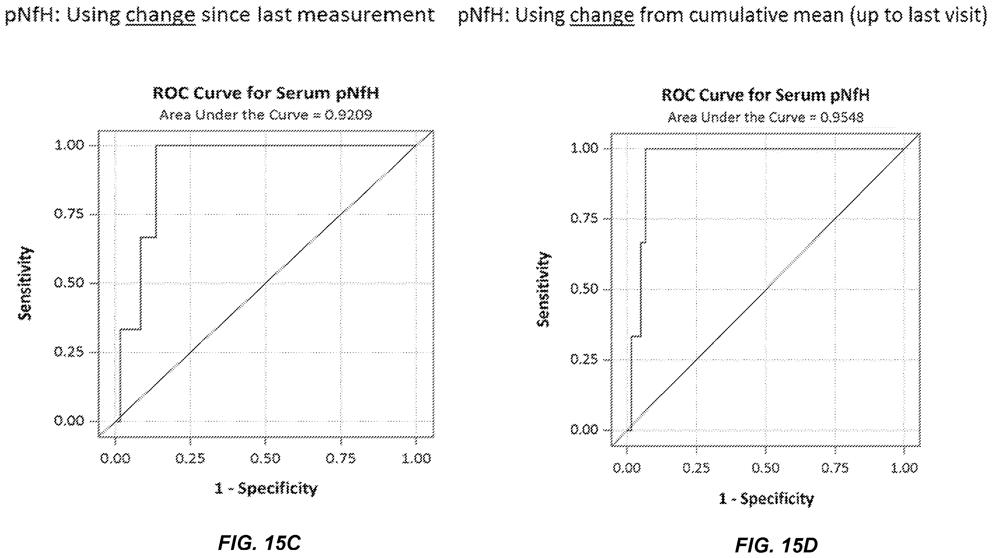

**Figures 15A-15D****: Receiver operating characteristic (ROC) analysis of the predictive utility of the change in serum NfL (****Figure 15A****).** The change in serum NfL since the last measurement (compared to the value measured at the prior visit) is used to predict the likelihood of phenoconversion (development of clinically manifest disease) within the following year (AUC= 0.9625). **ROC analysis of the predictive utility of the change in serum NfL (****Figure 15B****).** The change in serum NfL from the cumulative mean (up to last visit) is used to predict the likelihood of phenoconversion (development of clinically manifest disease) within the following year (AUC= 0.9625). **ROC analysis of the predictive utility of the change in serum pNfH (****Figure 15C****).** The change in serum pNfH since the last measurement (compared to the value measured at the prior visit) is used to predict the likelihood of phenoconversion (development of clinically manifest disease) within the following year (AUC= 0.9209). **ROC analysis of the predictive utility of the change in serum pNfH (****Figure 15D****).** The change in serum pNfH from the cumulative mean (up to last visit) is used to predict the likelihood of phenoconversion (development of clinically manifest disease) within the following year (AUC= 0.9548).

### DETAILED DESCRIPTION

The disclosure provides a method of predicting the onset of clinical manifestations of amyotrophic lateral sclerosis (ALS) in a pre-symptomatic human subject. The method involves measuring neurofilaments, neurofilament light chain (NfL) and/or phosphorylated neurofilament heavy chain (pNfH) levels, from a pre-symptomatic subject as markers to predict the onset of clinical manifestations of ALS (i.e., when a subject, who may be at risk for ALS, will develop clinical symptoms of ALS). Put another way, the disclosure provides a method for determining when the clinical manifestation of ALS will occur. The detection of increased levels of pNfH and/or NfL from a pre-symptomatic human subject signals the appearance of clinically manifest ALS, which enables the identification of subjects for ALS treatment prior to developing symptoms. The disclosure is the first to identify when, in the natural progression of disease, pNfH and NfL are elevated in the blood and CSF of subjects not yet demonstrating symptoms of the disease, but who will eventually develop clinical ALS. "Clinical manifestations of ALS" or "clinically manifest ALS" refer to the combination of an individual's symptoms (complaints) and overt physical signs which become manifest in the neurological examination which lead to the state of clinically manifest ALS.

The described embodiments illustrate representative examples of methods of the disclosure. From the description of these embodiments, other aspects of the disclosure can be made and/or practiced based on the description provided below. The methods involve use of immunological and molecular biological techniques described in methodology treatises such as Current Protocols in Immunology, Coligan et al., ed., John Wiley & Sons, New York. Techniques of molecular biology are described in detail in treatises such as Molecular Cloning: A Laboratory Manual, 2nd ed., vol. 1-3, Sambrook et al., ed., Cold Spring Harbor Laboratory Press, Cold Spring Harbor, N.Y., 2001; and Current Protocols in Molecular Biology, Ausubel et al., ed., Greene Publishing and Wiley-Interscience, New York. General methods of medical treatment are described in McPhee and Papadakis, Current Medical Diagnosis and Treatment 2010, 49th Edition, McGraw-Hill Medical, 2010; and Fauci et al., Harrison's Principles of Internal Medicine, 17th Edition, McGraw-Hill Professional, 2008.

### Neurofilaments

Neurofilaments are cytoskeletal intermediate filaments specific to neurons and have been extensively studied as potential biomarkers in neurological disorders (Turner et al., Lancet Neurol 2009;8:94-109). In various embodiments, the method comprises measuring the level of pNfH and/or NfL protein, preferably pNfH and NfL protein. Optionally, the method comprises measuring pNfH and/or NfL protein in blood serum, plasma, and/or cerebrospinal fluid (CSF) of the subject. pNfH and NfL protein levels are measured using any suitable method, such as antibody-based immunoassays. For example, neurofilament light chain (NfL) is readily detected by antibody-based immunoassays (Boylan et al., J Neurochem 2009*;* Boylan et al. J Neurol Neurosurg Psychiatry 2012; Brettschneider et al., Neurology 2006;66:852-856; Gaiottino et al. PLoS One 2013;8:e75091; Ganesalingam et al., Amyotroph Lateral Scler Frontotemporal Degener 2013;14:146-149; Lu et al., J Neurol Neurosurg Psychiatry 2014; Tortelli et al., Eur J Neurol 2012; Zetterberg et al., Eur J Neurol 2007;14:1329-1333). Phosphorylated neurofilament heavy (pNfH) is also readily detected by antibody based immunoassays. It will be appreciated that antibodies for use in the methods of detection or quantification may be monoclonal or polyclonal, and may be obtained by subtractive techniques. Examples of antibodies are described in, e.g. Lu et al., J Neurol Neurosurg Psychiatry 2015; 86(5): 565-5730; Lu et al., Neurology 2015; 84(22): 2247-2257. All measurements of neurofilament heavy (NfH) described herein, refer to phosphorylated neurofilament heavy (pNfH).

In various embodiments, NfL levels may be detected using the Simoa NF-light^{®} assay (Quanterix). The Simoa NF-light^{®} assay is a digital immunoassay for the quantitative determination of NF-L in serum, plasma and CSF (Kuhle et al., Clin Chem Lab Med 2016 2015-1195).

In various embodiments, the method involves measuring the levels of neurofilament aggregates, or cleavage products of NfL and/or NfH. pNfH may be hyperphosphorylated or hypophosphorylated, and the sample may comprise pNfH differentially phosphorylated to varying degrees. Techniques for detecting aggregates, or cleavage products of pNfH and/or NfL are described in, for example, Lu et al., J Neurosci Methods. 2011;195(2):143-50*;* Lu et al., J Neurol Neurosurg Psychiatry. 2015;86(5):565-73; and Puentes et al., J Neurol Neurosurg Psychiatry 2014;85 (3):274-278.

In various aspects, protein levels of pNfH and NfL are compared to protein levels of pNfH and NfL (optionally from blood serum, plasma, and/or CSF) of healthy controls or previously measured levels in the subject. The levels of neurofilament aggregates, or the levels of cleavage products of pNfH and/or NfL in a sample (e.g., blood serum and/or CSF) of subjects at risk of developing ALS are optionally compared to levels in healthy controls or levels previously measured in the subject. Increased levels of pNfH and NfL in subjects signal the onset of clinical manifestation of ALS (e.g., onset of clinical manifestations within 12-16 months).

### Phenoconversion

The progression of ALS, including transitions from the at-risk state to the disease state; from the pre-symptomatic to the symptomatic phase of disease; and from the pre-manifest stage to the prodromal stage of pre-symptomatic disease, are illustrated using a grayscale gradient to reflect the likelihood that these states, phases and stages exist along a continuum **(****Figure** 1). Referring to Figure 1, the vertical lines demarcating separation between states, phases and stages, are dotted to indicate the uncertainty in demarcating the timing of these milestones. The uncertainty arises in part from the reality that observations or measurement are not made continuously, and in part from the subjectivity of reporting and observation. The earliest biomarker evidence of disease, for example, may vary depending on when the biomarker is measured as well as the sensitivity of the biomarker to detect disease; this variability is reflected by the shadowed-arrows. Similarly, the timing of 'possible symptoms or signs' will depend on the subjectivity of the individual who reports the symptoms or the timing of careful neuromuscular and cognitive/behavioral examinations. 'Definite symptoms or signs' is typically an imputed time point and reflects the timing of onset of symptoms that an experienced evaluator understands to indicate the emergence of disease. Timing of diagnosis is also variable, but in studies of pre-symptomatic disease, typically occurs much sooner after symptom onset than occurs in clinical practice (this variability is also reflected by a shadowed-arrow). The pre-manifest stage encompasses the period prior to the appearance of the first suspicious (but nonspecific) manifestation of disease (e.g., a subjectively reported symptom), an asymptomatic sign elicited on neurological examination, or an EMG abnormality (e.g., isolated ongoing denervation changes in a single muscle) that could represent early disease but is insufficiently specific to make a diagnosis of ALS. Phenoconversion is the term used to define the transition from the pre-symptomatic to the symptomatic phase of disease. Phenoconversion is operationally defined as the first emergence of definite signs or symptoms of disease. Phenoconversion is not synonymous with diagnosis since the former may be reported, for example, by telephone whereas the latter typically requires an in-person assessment with physical examination and perhaps EMG. Phenoconversion also is sometimes considered as the first point in time at which an individual meets minimum published criteria for a diagnosis of ALS (i.e., evidence for progressive upper or lower motor neuron findings in at least one body region). The manifest period follows phenoconversion, and the prodrome is the period between possible symptoms/signs and phenoconversion.

As disclosed herein, as subjects approach the onset of ALS disease manifestation (i.e., the time of phenoconversion), levels of pNfH and NfL increase (i.e., increase compared to previous levels in the same subject or increased compared to levels of pNfH and NfL in healthy subject controls). Thus, in various aspects, the increased levels of pNfH and/or NfL are relative to levels of pNfH and/or NfL measured in the human earlier in time. In this regard, the method optionally comprises (a1) measuring pNfH and/or NfL levels in a subject at a first time point; (a2) measuring pNfH and/or NfL levels in the subject at a second time point; and (a3) comparing the levels of pNfH and/or NfL from (a1) and (a2). The first and second time points are distinct in time, and can be separated by days, weeks, months, or years. In various aspects, the first time point and the second time point are about three to about six months apart. The disclosure contemplates methods wherein the pNfH and/or NfL levels are measured multiple times (i.e., more than twice), optionally at somewhat regular intervals (e.g., about three to about six month intervals). Measurements may be repeatedly taken over an extended period of time, such as one, two, five, or ten years or the lifetime of the subject.

Alternatively, the method comprises detecting at least 24 pg/ml NfL in the sample (e.g., blood serum, plasma and/or cerebrospinal fluid (CSF)) of the subject. In various aspects, the method comprises detecting at least 33 pg/ml NfL. In this respect, multiple measurements of NfL need not be taken, but the disclosure contemplates embodiments comprising measuring NfL levels at multiple time points. In various aspects, as described further herein, subjects with NfL levels of 24 pg/mL or 33 pg/mL or more may begin ALS therapy. It will be appreciated that, depending on the particular ALS therapy and the circumstances of a particular subject, the NfL level threshold may be adjusted.

### Pre-symptomatic human ALS-associated gene mutation carrier

In various aspects of the disclosure, the human subject is a pre-symptomatic human ALS-associated gene mutation carrier. A pre-symptomatic human, as described herein, is a subject that shows no clinical evidence of ALS (i.e., insufficient signs or symptoms to be confident that ALS has become clinically apparent). A number of inherited conditions increase the risk or likelihood to developing ALS. A pre-symptomatic human ALS-associated gene mutation carrier is a human comprising one or more genetic mutations associated with ALS development. For example, the pre-symptomatic human ALS-associated gene mutation carrier comprises a mutation in one of more of the following genes: *superoxide dismutase 1* (SOD1), *chromosome 9 open reading frame* 72 (C9orf72). *fused in sarcoma* (FUS), *TAR DNA Binding Protein* (TARDBP or TDP-43), *valosin-containing protein* (VCP), *Angiogenin* (ANG), *Coiled-coil-helix-coiled-coil-helixdomain containing 10* (CHCHD10), *Chromatin modifying protein 2B* (CHMP2B), *Heterogenous nuclear ribonucleoprotein A*1(HNRNPA1), *Matrin 3* (MATR3), *Optineurin* (OPTN), *Profilin 1* (PFN1), *Spatacsin* (SPG11), *Sequestosome 1* (SQSTM1), *TDP-43* (TARDP), *TANK-binding kinase 1* (TBK1), *Tubulin Alpha* 1 (TUBA4A), *Ubiquilin-2* (UBQLN2) (Rowland and Shneider, N Engl J Med. 2001;344:1688-1700; Kwiatkowski et al., Science. 2009;323:1205-1208; Rosen et al., Nature 1993;362:59-62; Sreedharan et al., Science 2008;319:1668-1672; Vance et al., Science 2009;323:1208-1211; Johnson, J. O., et al., Neuron 2010;68, 857-864; Haeusler et al., Nat Rev Neurosci. 2016;17(6):383-95; Familial ALS Resource Booklet, The ALS Association 2018, www.alsa.org).

### ALS therapy

It is generally believed that the pathobiology of ALS begins focally within the nervous system and spreads, via an unknown mechanism, to become more generalized over time (Ravits et al., Neurology 2007;68:1576-1582; Ravits et al., Neurology 2007;68:1571-1575; Ravits et al., Neurology 2009;73:805-811). There are also reasons to believe that the disease process, after initiation, may become self-propagating, for example, by activation of a neuro-inflammatory response (Ransohoff et al., Science 2016;353:777-783). It is intrinsically easier to prevent or limit motor neuronal degeneration than to effect regeneration of motor neurons that have already died, which would require that these motor neurons extend axonal processes over long distances to re-establish connections with distant musculature. It is advantageous to begin treatment in subjects prior to, or as soon as possible after, phenoconversion begins. Yet, there are inherent challenges to medicating pre-symptomatic individuals, including the need for prolonged therapy with potential for toxicity or unwanted side effects. The method described herein allows the identification of subjects that will phenoconvert, and provides insight as to when symptoms will manifest. Thus, ALS treatment can be initiated to achieve a beneficial effect in the subject.

In various aspects of the disclosure, the method further comprises administering an ALS therapy (or an experimental therapy) to a subject. An ALS therapy includes, but is not limited to, one or more of the following: edaravone (Radicut^{®}), riluzole (Rilutek^{®}, Teglutik^{®}), mesenchymal stem cells, copper-ATSM (CuATSM), AB-1010 (masitinib), CK-2017357(tirasemtiv), E0302 (mecobalamin), NP001, pyrimethamine, ibudilast, glial restricted progenitor cells, mexiletine, memantine, GDC-0134, EPI-589, RNS-60, pimozide, tocilizumab, ezogabine, ODM-109, low-dose IL-2, amylyx, an antisense oligonucleotide, or gene therapy.

In one aspect, the antisense oligonucleotide targets one or more genetic mutation(s) associated with ALS. Antisense oligonucleotides for use in the instant disclosure include, for example, ISIS-SOD1Rx (BIIB067) (Clinical trial NCT02623699), C9orf72 (Sareen et al., Sci Transl Med. 2013;5(208):208ra149), ASO816 (Sareen et al., Sci Transl Med. 2013;5(208):208ra149), ASO061 (Sareen et al., Sci Transl Med. 2013;5(208):208ra149), ISIS SMNRx, ISIS 333611 (Miller et al., Lancet Neurol. 2013;12(5):435-42), or combinations thereof.

In various embodiments, gene therapy targets one or more genetic mutation(s) associated with ALS. Gene therapy refers to the introduction of exogenous nucleic acids to a subject in need thereof, such that the exogenous nucleic acids mediate production of therapeutic products. In various aspects, a viral vector (e.g., a retroviral vector) is employed to introduce an exogenous nucleic acid into cells affected by the defective gene, e.g., motor neurons involved in ALS. The exogenous nucleic acid encodes a gene product that complements a defect associated with ALS, such as (but not limited to) SOD1, C9orf72, FUS, TARDBP, VCP, or combinations thereof. Numerous vectors useful for this purpose are described in the art (Miller, Human Gene Therapy 15-14, 1990; Moen, Blood Cells 17:407-416, 1991; Le Gal La Salle et al., Science 259:988-990, 1993). An example of a viral vector-mediated gene therapy for use in the context of the method is VY-SOD101. VY-SOD101 is composed of a adeno-associated virus (AAV) capsid and transgene with a micro RNA (miRNA) expression cassette that knock-downs the production of SOD1 mRNA. *(Voyager Therapeutics Announces Lead Clinical Candidate Selection for Monogenic Form of Amyotrophic Lateral Sclerosis (ALS).* Press release retrieved from ir.voyagertherapeutics.com).

In related embodiments, the ALS therapy is administered within 12-16 months of detecting an increase in pNfH and/or NfL levels in the subject. The ALS therapy may be administered 0, 1, 2, 3, 4, 5, 6, 7, 8, 9, 10, 11,12, 13, 14, 15, or 16 months post-detection of elevated levels of pNfH and/or NfL.

In various aspects, the disclosure provides a method comprising (a) measuring phosphorylated neurofilament heavy chain (pNfH) and/or neurofilament light chain (NfL) levels in a pre-symptomatic human ALS-associated gene mutation carrier at two or more time points; and (b) administering an ALS therapy to the subject when (i) absolute NfL levels are at least 24 pg/ml and/or (ii) pNfH and/or NfL levels increase between the measurements taken at two or more time points in step (a). Optionally, step (b) comprises administering an ALS therapy to the subject when absolute NfL levels are at least 24 pg/ml. Alternatively, step (b) comprises administering an ALS therapy to the subject when pNfH and/or NfL levels increase between the measurements taken at two or more time points in step (a) (e.g., two or more time points about three to about six months apart).

The terminology used herein is for the purpose of describing particular embodiments only and is not intended to be limiting. Additional aspects and details of the disclosure will be apparent from the following examples, which are intended to be illustrative rather than limiting.

### EXAMPLES

### General methods

*Sample Collection, Processing, and Storage.* For serum analysis, blood was collected in a red top BD vacutainer and allowed to clot upright at room temperature for 1-2 hours. Following centrifugation (1750g for 10 minutes at 4°C) serum was aliquoted into cryogenic sterile freestanding conical microtubes (Nalgene or Bio Plas Inc.) and quickly stored at -80°C until use. CSF (free of macroscopic hemoglobin) was collected in polypropylene tubes, centrifuged (1750g for 10 minutes at 4°C), aliquoted using a sterile pipette into pre-capped polypropylene cryogenic sterile freestanding conical microtubes, frozen within 15-20 minutes of collection, and stored at -80°C until use.

*Neurofilament Light.* The protocol for quantification of NfL is based on a electrochemiluminescence (ECL) immunoassay (Gaiottino et al. PLoS One 2013;8:e75091; Lu et al. Neurology 2015; 84(22):2247-57). Briefly, 96-well plates (Multi-Array^{®} plates, Meso Scale Discovery (MSD), Gaithersburg, MD) including integrated screen-printed carbon ink electrodes on the bottom of the wells are used. All samples were evenly distributed on each plate and measured in duplicate at the same dilution. All NfL assays were performed blind to group/disease state. Each plate contained calibrators (0-10,000 pg/mL) and quality controls; if required, samples were appropriately diluted to fall in the range of the standard curve. Inter-assay coefficients of variance are below 10% and the mean intra-assay coefficients of variance are below 10%. Linearity of the NfL was established (0-50,000 pg/mL) as previously reported (Gaiottino et al. PLoS One 2013;8:e75091; Lu et al. Neurology 2015; 84(22):2247-57).

*Statistical Analysis.* NfL concentrations that were below the level of detection were excluded from the summary statistics in Table 2, but included in analyses using an imputed value of 0.37 pg/ml, which was the lowest measured value in our data. Due to the highly skewed distribution of NfL concentrations and CSF/serum ratios, and the presence of outliers, analyses were performed using non-parametric tests or natural logarithm-transformed values. For ease of interpretation of log-transformed values, a small constant of 0.63 (=1-0.37) was added to NfL values before taking the natural logarithm, so that the lowest log-transformed value is 0 (rather than a negative value). In all cases the logarithm (log) scale refers to the natural logarithmic scale. For CSF NfL, one extreme outlier was excluded. Summary statistics for numerical variables are provided in mean±SD (or, for variables with a highly skewed distribution, in median and range), and for categorical variables in frequency and percentage. For phenoconverters in *Pre-fALS,* date of onset is determined based on the earlier of: unequivocal symptoms reported by the participant, or subclinical signs of disease detected through detailed neuromuscular examination or EMG that clearly indicate disease. All 10 phenoconverters met the former definition. Date of onset of affected individuals was based on participants' recollection and self-report. Spearman rank correlation, Wilcoxon rank-sum test or Kruskal-Wallace test, and regression analyses were employed in the analysis of cross-sectional data. Mixed model analysis (with random intercept, unstructured covariance structure, and Kenward-Roger degrees of freedom) were employed in the analysis of longitudinal data. For the Spearman correlation of serum and CSF over follow-up, to correct for the within-person correlation, a bootstrap percentile confidence interval was computed by stratified sampling (with replacement) within the 4 participant groups. The level of statistical significance was set at 0.05 (two-sided); given the discovery and exploratory nature of this study, however, the focus of the analyses were less on statistical testing but more on characterizing the NfL concentrations observed. All statistical analyses were performed and graphics produced using SAS 9.4 (SAS Institute Inc., Cary, NC, USA).

### Example 1: Pre-Symptomatic Familial ALS (Pre-fALS) Cohort and Phenoconversion

Pre-Symptomatic Familial ALS (Pre-fALS) is a longitudinal natural history and biomarker study of individuals across North America who are at genetic risk for developing ALS but who, at the time of enrollment, demonstrate no clinical evidence of disease and are not yet affected with ALS (Benatar, et al., Neurology 2012;79(16):1732-1739; Benatar et al., Muscle & nerve 2016;53:169-182; NCT00317616). The study population comprised of pre-symptomatic individuals (English-speaking, recruited from across North America) who are carriers of any ALS-associated gene mutation (in SOD1, C9orf72, TARDBP, FUS, VCP, etc.), the only population known to be at risk for ALS.

In addition to assaying systemic markers of neurodegeneration (e.g., p75 neurotrophin receptor extracellular domain, and poly(GP), neurofilament light and neurofilament heavy) in relevant biological fluids (urine, blood and CSF). electrophysiological techniques such as electrical impedance myography (EIM) and quantitative motor testing were used to study each region of motor domain (i.e., bulbar, cervical thoracic and lumbosacral) individually. An array of neuropsychological tests may are used to quantify aspects of cognitive and behavioral function that are known to reflect frontotemporal dysfunction and which are characteristic of the subset of ALS patients in whom frontotemporal spectrum pathology emerges (**Table 1**).

**Table 1. Biomarkers Utilized to Study Pre-Symptomatic Disease**

| | **Frontotemporal** | **Bulbar** | **Cervical** | **Thoracic** | **Lumbosacral** |
|---|---|---|---|---|---|
| **Clinical** | Neuropsych testing | Clinical exam | Clinical exam | Spirometry | Clinical exam |
| **Electrophysiology** | --- | EMG, EIM | EMG, EIM, MUNE | EMG, EIM | EMG, EIM |
| **Motor testing** | --- | Q-motor | Q-motor | --- | Q-motor |
| **Neuroimaging ¹** | T1, DTI, MRS | T1, DTI, MRS | T1, DTI, MRS | --- | T1, DTI, MRS |
| **Biological fluids (Systemic):** Urine (p75), serum (NfL, pNfH), CSF (NfL, pNfH, poly-GP), PBMC (poly-GP). | | | | | |

| | | | | | |
|---|---|---|---|---|---|
| ¹ Advanced neuroimaging is performed in eligible *Pre-fALS* study participants, but is excluded from the scope of this grant due to budgetary constraints. EIM = electrical impedance myography. EMG = electromyography. MUNE = motor unit number estimation. Q-motor = Quantitative motor testing. T1 = T1 structural imaging. DTI = diffusion weighted imaging. MRS = magnetic resonance spectroscopy. p75 = p75 neurotrophin receptor extracellular domain. NfL = neurofilament light chain. pNfH = phosphorylated neurofilament heavy. poly-GP = poly-Glycine-Proline. PBMC = peripheral blood mononuclear cell. | | | | | |

To date, a 347 person-years follow-up has been conducted among the 100 gene mutation carriers enrolled in the Pre-fALS Cohort. With 11 individuals having phenoconverted to manifest disease, the two-year phenoconversion rate (based on Kaplan-Meier estimate) across all genetic mutations is approximately 9% (**Figure 2**). Since knowledge of SOD1 as a cause of ALS has been available for a longer period of time than knowledge of other ALS-causing genetic mutations, the Pre-fALS Cohort was dominated by SOD1 mutation carriers for many years (accounting for 280 person-years follow-up). With the more recent discovery of the C9orf72 hexanucleotide repeat expansion mutation as the most common genetic cause of ALS, enrollment has been focused more on this population, and to date has accumulated ~50 person-years follow-up in this group. The remaining 10 person-years follow-up that has been accumulated is spread across individuals with mutations in TARDBP, FUS and VCP. Based on these preliminary data, it is estimated that the two-year phenoconversion rate among all mutation carriers is ~9%, and among the SOD1 A4V mutation carriers ~20% (**Figure 2**).

### Example 2: Serum neurofilament light levels are elevated among the phenoconverters and ALS patients

NfL and pNfH have been reported to be elevated in blood and CSF of patients with ALS relative to controls, but *when* in the natural history of disease these biomarkers increase has, until now, been unknown. In cross-sectional analysis, serum NfL is significantly (p<0.001) elevated among ALS patients compared to healthy controls and pre-symptomatic individuals who have not yet phenoconverted. Using the patient samples collected in the Pre-fALS Cohort study described in Example 1, NfL levels were measured in the serum of 34 controls, 84 pre-symptomatic gene mutation carriers, 11 phenoconverters (with samples collected both before and after phenoconversion) and 17 affected (i.e., patients with ALS at the time of initial assessment); the affected group included 11 ALS patients in whom there is no identified genetic mutation (**Figure 3** **and** **Figures 5A****-5D**). The median delay from time of onset to diagnosis was 16.4 months (range 2.6-83.8) among ALS patients, and notably shorter, 1.8 months (range 0.5-5.5) among the converters. Cross-sectional clinical and NfL data are available from all participants at time of initial sample collection ("baseline"), with longitudinal data available from a subset of 10 controls, 51 at-risk, 8 converters, and 11 affected. Overall, clinical and NfL data were available from 343 person-visits. The at-risk group largely comprised individuals with an SOD1 mutation (n=28 [54%] A4V, n=24 [46%] non-A4V) or a C9orf72 HRE (n=27 [32%]); and all but one of the converters were SOD1 mutation carriers. EMG (of both arms and legs, mid-thoracic paraspinals and bulbar musculature) was normal in all phenoconverters prior to phenoconversion, with the exception of the FUS phenoconverter, in whom there were nonspecific changes restricted to L5 innervated muscles 9 months prior to phenoconversion.

The control and at-risk groups are comparable in baseline age (mean±SD = 46±11 and 45±12 years, respectively), with the converters slightly older (50±12 years) and the affected about 10 years older (60±8 years; p<0.01 when compared to controls or at-risk). As of July 2016, the cut-off date for the NfL experiments reported here, we had conducted a median of 2-3 (and up to 5-7) longitudinal visits on the N=80 participants in the longitudinal subset, with varying lengths of follow-up for the different groups.

Using longitudinal analysis, data from pre-symptomatic gene mutation carriers were followed longitudinally from the pre-symptomatic state and through the time of phenoconversion (the development of clinically manifest disease). Levels of NfL in healthy controls (**Figure 5A**) and pre-symptomatic gene mutation carriers (**Figure 5B**) were generally below 4 on a natural logarithmic scale (shown as a faint horizontal dotted line) and largely stable over time. As the at-risk subjects approach the onset of manifest disease (i.e., the time of phenoconversion), levels began to rise (**Figure 5C**). By the time of diagnosis, levels of NfL were generally above 4 (per natural log scale) and remain largely stable as disease progressed (**Figure 5D**). These data shed light on the temporal course of the rise in serum NfL levels, suggesting that levels begin to increase in the ~12 months prior to manifest disease. Although not shown, there was a strong correlation (r = 0.9, p<0.001) between serum and CSF NfL (available from fewer study participants since not everyone undergoes LP at each visit), and the concentration of CSF NfL also increased in advance of the appearance of manifest disease.

These data provide the first ever insight into the longitudinal trajectory of CSF and blood neurofilament levels quantified in the same individuals both before and around the time of phenoconversion to clinically manifest disease. These data show that neurofilament levels rise within the 12-months prior to phenoconversion, providing evidence that CSF and blood neurofilament levels may be used both as a biomarker of pre-symptomatic disease and as a prognostic marker, predicting the short-term likelihood of developing clinically manifest disease.

### Relationship between serum and CSF NfL Levels

Serum and CSF NfL levels are highly correlated: including only cross-sectional data at first CSF collection (total N=85 participants across all 4 groups), Spearman rank correlation r=0.65, p<0.0001; and including all available data (total 155 person-visits across all 4 groups), r=0.70, 95% CI 0.60-0.78. NfL concentrations in CSF are invariably higher than serum by 8- to 286-fold (median 57-fold). Specifically, the CSF/serum ratios of NfL were similar in controls (18 person-visits, mean ± SD = 66 ± 48), at-risk (110 person-visits, 65 ± 52), and converters (10 person-visits, 67 ± 27), and slightly higher in affected (16 person-visits, 77 ± 28). Moreover, the CSF/serum ratio remained largely stable over time among the N=49 participants with longitudinal CSF and serum data (slope estimate on the natural logarithm scale = -0.059, 95% CI [-0.150, 0.032]) (**Figure 9**).

Elevated levels of NfL observed in the at-risk subjects is used to determine the onset of ALS and predict the timing of the appearance of clinically manifest disease. The ALS therapy (or experimental therapy) is, in various aspects, administered within 12-16 months (e.g., within 12 months, within 13 months, within 14 months, within 15 months, or within 16 months) post detection of elevated levels of NfL to prevent, delay, or treat ALS.

### Cross-Sectional NfL Concentration at Baseline

Baseline NfL levels in serum (**Table 2**) were comparable between controls (median [range] = 9 [2-33] pg/ml) and at-risk individuals (13 [1-62] pg/ml), irrespective of genotype (**Figure 4A-4B**). On the other hand, serum levels were higher in patients with ALS (121 [21-555] pg/ml) compared to controls (Wilcoxon rank-sum test, p<0.0001) and at-risk (p<0.0001). While lumbar puncture was only performed at 155 of 343 (45%) person-visits, a similar data pattern was observed in CSF NfL (**Table 2,** **Figures 4C-4D**).

### Longitudinal Changes in NfL Concentration

Longitudinally, serum NfL levels are essentially stable in controls and ALS patients (**Figures 6A-6D** **and** **7B**), although there are as yet insufficient data to reliably estimate the slopes in a mixed model analysis. By contrast, in at-risk individuals, adjusting for baseline age, serum NfL increases by an average of 2.41pg/ml per 10-year increase in age (p=0.004). Moreover, among converters, elevated NfL levels (i.e., above the highest value seen in controls) were observed as far back as 11.6 months prior to phenoconversion, and their NfL levels continued to increase through at least the first 6 months after symptom onset (**Figures 6C** **and** **7A**). While CSF was not available from all participants at every time point, a similar pattern was seen in CSF NfL. Noteworthy are two other observations: (a) all available serum NfL measured >1 year prior to phenoconversion were within the range of controls; and (b) all available serum NfL measured after phenoconversion were well above the range observed among controls.

### Effects of Age, Sex, and Gene Mutation on NfL Concentration

In cross-sectional analysis among n=34 controls, there is a modest correlation between age and baseline serum NfL (Spearman rank correlation, r=0.32, p=0.065), with an average increment of 1.39 pg/ml for every decade increase in age. While, thus far, too few control participants with sufficiently long follow-up duration were included to reliably estimate the annual increase over time, the cross-sectional data suggest that the magnitude of the age-related increase in serum NfL is negligible considering the levels observed in the affected group (**Figures 6A-6D** **and** **8**) and converter group (**Figures 6A-6D** **and** **7A-7B**). A scatterplot of baseline serum NfL levels versus baseline age (Figure 8) showed that, although NfL levels were slightly higher among older individuals in the control and at-risk groups, the magnitude of the increase is negligible in comparison to the levels of NfL observed among ALS patients (i.e., older age did not explain the increase in serum NfL among ALS patients).

Furthermore, baseline serum and CSF NfL levels did not differ by sex (p>0.16 across the 4 groups; **Table 3**) or by gene mutation (at-risk group only, SOD1 A4V vs. SOD1 non-A4V vs. C9orf72 vs. other gene mutations, p=0.12 for serum and 0.52 for CSF; **Tables 4 and 5**).

### Example 3: Serum neurofilament phosphorylated heavy levels are elevated among the phenoconverters and ALS patients

As with neurofilament light, levels of neurofilament phosphorylated heavy (pNfH) were measured in the serum and CSF of pre-symptomatic gene mutation carriers, phenoconverters (with samples collected both before and after phenoconversion), patients with ALS and healthy (non-gene mutation carrier) controls.

Levels of pNfH in the blood serum and CSF of healthy controls, pre-symptomatic gene mutation carriers and ALS patients were compared. Comparable to data with NfL, baseline pNfH levels in serum (**Figures 10A-10B**) were comparable between controls and pre-symptomatic individuals. On the other hand, serum levels were higher in patients affected with ALS compared to controls (**Figures 10A-10B**). Similar data was obtained for CSF pNfH (**Figures 10C-10D**).

### Longitudinal Changes in pNfH Concentration

Longitudinally, serum pNfH levels are essentially stable in controls and ALS patients (**Figures 11A-11D**), although there are as yet insufficient data to reliably estimate the slopes in a mixed model analysis.

As shown Figure 11C, as the at-risk subjects approach the onset of manifest disease (i.e., the time of phenoconversion), pNfH levels in phenoconverters are increased compared to levels at earlier time points, and the increased levels are detectable before they convert.

In addition, when comparing pNfH and NfL levels in both serum and CSF, there was found to be a correlation between natural log of serum pNfH and serum NfL (**Figure 12**) and a correlation between natural log of CSF pNfH and CSF NfL (**Figure 13**).

Elevated levels of pNfH observed in the at-risk subjects are used to determine the onset of ALS. The ALS therapy is optionally administered within 12 months post detection of elevated levels of pNfH to prevent, delay or treat ALS.

### Example 4: Absolute levels of NfL and the change in serum NfL and pNfH levels for predicting the onset of ALS

Receiver operating characteristic (ROC) curve analysis was employed to assess the value of the absolute levels of NfL and pNfH or changes in levels in predicting phenoconversion (i.e. progression from the asymptomatic/at-risk state to the state of having ALS). In **Figure 14****,** the absolute value of serum NfL (i.e., using raw value from the last measurement) were used to predict the likelihood of phenoconversion (development of clinically manifest disease) within the following year. In **Figure 15A****,** the change since the last measurement (from previous visit/measurement) in serum NfL values was used to predict the likelihood of phenoconversion (development of clinically manifest disease) within the following year. In **Figure 15B****,** the change in serum NfL from the cumulative mean (up to last visit) was used to predict the likelihood of phenoconversion (development of clinically manifest disease) within the following year. In **Figure 15C****,** the change in serum pNfH since the last measurement (compared to the value measured at the prior visit) was used to predict the likelihood of phenoconversion (development of clinically manifest disease) within the following year. In **Figure 15D****,** the change in serum pNfH from the cumulative mean (up to last visit) was used to predict the likelihood of phenoconversion (development of clinically manifest disease) within the following year. For each ROC analysis, area under the curve (AUC) analysis was performed, where the closer the AUC value is to 1.0, the better the prediction.

The ROC analysis of the predictive utility of the absolute values of serum NfL revealed that the absolute value of serum NfL was predictive of the likelihood of phenoconversion (AUC of 0.8780; **Figure 14**). The data in **Figure 14** suggest that an absolute NfL value of at least 33.58 pg/mL is predictive of phenoconversion. In subjects with NfL levels above this threshold, practitioners may consider administering ALS therapy.

The ROC analysis of the predictive utility of both the change in values of serum NfL from last measurement and the change from the cumulative mean revealed that the change in serum NfL levels was highly predictive of the likelihood of phenoconversion (AUC of 0.9625; **Figure 15A and Figure 15B**). The data in **Figure 15A** and/or **Figure 15B** suggest that a change in the NfL value (from the last measurement or change from the cumulative mean) of ~10 pg/ml is predictive of phenoconversion. In subjects with changes in NfL levels of about 10 pg/mL or more, practitioners may consider administering ALS therapy.

The ROC analysis of the predictive utility of both the change in values of serum pNfH from last measurement and the change from the cumulative mean revealed that the change in serum pNfH levels were highly predictive of the likelihood of phenoconversion (AUC of 0.9209 **Figure 15C****;** and AUC of 0.9548 for **Figure 15D**). The data in **Figure 15C** and/ or **Figure 15D** suggest that a change in the pNfH value (from the last measurement or change from the cumulative mean) of ~14 pg/ml or ~17 pg/ml, respectively, is predictive of phenoconversion. In subjects with changes in pNfH levels of about 14 pg/mL or about 17 pg/mL or more, practitioners may consider administering ALS therapy.

In each case the AUC value for the change in values of serum NfL and pNfH levels was above 0.9 (i.e., correctly predicts whether someone will develop ALS within a year with 90% accuracy).

In conclusion, both the absolute value (for NfL levels) and the change in value (for both NfL and pNfH levels) may be used as biomarker for predicting who will develop ALS within the ensuing year.

**Table 2: Serum and CSF NfL concentration**

| | | Baseline Visit Only | | All Available Visits | |
|---|---|---|---|---|---|
| | | Serum NfL | CSF NfL | Serum NfL | CSF NfL |
| Control (N=34; total 56 visits) | | *N=32^{d}* | *N=15^{f}* | *53 visits ^{d}* | *18 visits* |
| Original scale (pg/ml) | Median (Range) | 9.0 (2.2-33.1) | 491 (306-1,573) | 10.7 (0.4-33.5) | 482 (306-2,460) |
| Log-transformed *^{a}* | Mean ± SD (Range) | 2.4 ± 0.7 (1.0-3.5) | 6.3 ± 0.6 (5.7-7.4) | 2.5 ± 0.7 (0.01-3.5) | 6.4 ± 0.6 (5.7-7.8) |

| At-Risk (N=84; total 213 visits) | | *N=75 ^{e}* | *N=50'* | *195 visits ^{e}* | *110 visits* |
|---|---|---|---|---|---|
| Original scale (pg/ml) | Median (Range) | 12.6 (1.1-62.4) | 641 (301-2,719) | 13.9 (1.1-75.6) | 681 (225-3,629) |
| Log-transformed *^{a}* | Mean ± SD (Range) | 2.5 ± 0.7 (0.6-4.1) | 6.5 ± 0.5 (5.7-7.9) | 2.7 ± 0.7 (0.6-4.3) | 6.6 ± 0.6 (5.4-8.2) |

| Converter (N=10 ^{b}; total 28 visits ^{c}) | | *N=10* | *N=3'* | *28 visits* | *10 visits ^{g}* |
|---|---|---|---|---|---|
| Original scale (pg/ml) | Median (Range) | 19.1 (6.9-104.3) | 2,558 (772-5,679) | 86.2 (6.9-720.4) | 7,664 (772-16,678) |
| Log-transformed *^{a}* | Mean ± SD (Range) | 3.1 ± 1.0 (2.0-4.7) | 7.7 ± 1.0 (6.6-8.6) | 4.3 ± 1.3 (2.0-6.6) | 8.6 ± 1.0 (6.6-9.7) |

| Affected (N=17; total 44 visits) | | *N=17* | *N=6* | *44 visits* | *16 visits* |
|---|---|---|---|---|---|
| Original scale (pg/ml) | Median (Range) | 120.5 (20.5-554.7) | 10,655 (2,142-14,964) | 99.0 (20.5-554.7) | 9,413 (2,142-21,658) |
| Log-transformed *^{a}* | Mean ± SD (Range) | 4.8 ± 0.8 (3.1-6.3) | 9.0 ± 0.8 (7.7-9.6) | 4.7 ± 0.7 (3.1-6.3) | 9.0 ± 0.8 (7.7-10.0) |

| | | | | | |
|---|---|---|---|---|---|
| Baseline = first visit at which serum sample was available (with or without contemporaneous CSF collection) N = number of participants. Visits = number of person-visits. *^{a}* Natural algorithm *^{b}* All 10 converters were pre-symptomatic at baseline. *^{c}* The 28 person-visits in the converter group included visits from when participants were pre-symptomatic and after phenoconversion. *^{d}* In the control group, serum NfL at 3 person-visits (2 baseline, 1 follow-up) were below the level of detection and not included in table above. *^{e}* In the pre-symptomatic group, serum NfL at 18 person-visits (9 baseline, 9 follow-up) were below the level of detection and not included in the summary statistics above. *^{f}* An additional N=1 control, N=6 at-risk, and N=3 converters had CSF NfL at follow up (but not baseline) visits. *^{g}* In the converter group, CSF NfL at 1 baseline visit was below the level of detection and not included in table above. | | | | | |

**Table 3. Baseline serum and CSF NfL levels, by sex**

| | **Control** | **At-Risk** | **Converter** | **Affected** |
|---|---|---|---|---|
| ***Baseline serum NfL, log-transformed^{a}, Mean ± SD:*** | | | | |
| **Female** | 2.29 ± 0.80 (N=19) | 2.19 ± 1.06 (N=53) | 3.18 ± 1.18 (N=6) | 5.07 ± 0.66 (N=8) |
| **Male** | 2.22 ± 0.95 (N=15) | 2.37 ± 1.07 (N=31) | 3.01 ± 0.78 (N=4) | 4.49 ± 0.73 (N=9) |

| ***Baseline CSF NfL, log-transformed^{a}, Mean ± SD:*** | | | | |
|---|---|---|---|---|
| **Female** | 6.15 ± 0.53 (N=8) | 6.44 ± 0.54 (N=33) | 3.92 ± 5.55 (N=2) | 9.00 ± 0.53 (N=8) |
| **Male** | 6.53 ± 0.56 (N=7) | 6.65 ± 0.46 (N=17) | 7.65 ± 1.41 (N=2) | 8.96 ± 1.12 (N=3) |

| | | | | |
|---|---|---|---|---|
| *^{a}* Natural logarithm | | | | |

**Table 4. Baseline serum and CSF NfL levels in At-Risk group, by genotype**

| | ***SOD1* A4V** | ***SOD1* nonA4V** | ***C9orf72* HRE** | **Other** |
|---|---|---|---|---|
| **Baseline serum NfL Log-transformed ^{a}, Mean ± SD** | 1.90 ± 1.12 (N=28) | 2.19 ± 1.30 (N=24) | 2.64 ± 0.63 (N=27) | 2.50 ± 0.63 (N=5) |
| **Baseline CSF NfL Log-transformed ^{a}, Mean ± SD** | 6.40 ± 0.46 (N=16) | 6.36 ± 0.38 (N=11) | 6.66 ± 0.65 (N=19) | 6.70 ± 0.22 (N=4) |

| | | | | |
|---|---|---|---|---|
| *^{a}* Natural logarithm | | | | |

**Table 5. Baseline serum NfL levels in ALS group, by genetic status**

| | ***Known Genotype (SOD1, C9orf72 HREM)*** | ***Unknown Genotype*** |
|---|---|---|
| **Baseline serum NfL Log-transformed ^{a}, Mean ± SD** | 5.15 ± 0.67 (N=6) | 4.56 ± 0.77 (N=11) |

| | | |
|---|---|---|
| *^{a}* Natural logarithm | | |

## Claims

1. A method of predicting the onset of clinical manifestations of amyotrophic lateral sclerosis (ALS) in a pre-symptomatic human subject, the method comprising
(a) measuring phosphorylated neurofilament heavy chain (pNfH) and/or neurofilament light chain (NfL) levels in a sample of a subject in a pre-symptomatic phase, and
(b) predicting the onset of ALS,
wherein increased levels of pNfH and/or NfL relative to levels of pNfH and/or NfL measured in the subject earlier in time signal the onset of clinical manifestations of ALS.

2. The method of claim 1, wherein step (a) comprises
(a1) measuring pNfH and/or NfL levels in a sample of the subject at a first time point, and
(a2) measuring pNfH and/or NfL levels in a sample of the subject at a second time point, and
(a3) comparing the levels of pNfH and/or NfL from (a1) and (a2).

3. The method of claim 2, wherein the first time point and the second time point are three to six months apart.

4. The method of claim 1, wherein step (b) comprises detecting NfL levels of at least 24 pg/ml.

5. The method of any one of claims 1-4, wherein the subject is a pre-symptomatic human ALS-associated gene mutation carrier.

6. The method of claim 5, wherein the pre-symptomatic human ALS-associated gene mutation carrier comprises a mutation in superoxide dismutase 1 (SOD1), chromosome 9 open reading frame 72 (C9orf72), fused in sarcoma (FUS), TAR DNA Binding Protein (TARDBP), valosin-containing protein (VCP), Angiogenin (ANG), Coiled-coil-helix-coiled-coil-helixdomain containing 10 (CHCHD10), Chromatin modifying protein 2B (CHMP2B), Heterogenous nuclear ribonucleoprotein A1(HNRNPA1), Matrin 3 (MATR3), Optineurin (OPTN), Profilin 1 (PFN1), Spatacsin (SPG11), Sequestosome 1 (SQSTM1), TDP-43 (TARDP), TANK-binding kinase 1 (TBK1), Tubulin Alpha 1 (TUBA4A), Ubiquilin-2 (UBQLN2), or a combination thereof.

7. The method of any one of claims 1-6, wherein step (a) comprises measuring the levels of neurofilament-containing hetero-aggregates, or cleavage products of pNfH and/or NfL.

8. The method of any one of claims 1-7, wherein the pNfH and NfL protein levels are measured by antibody-based immunoassays.

9. An ALS therapy for use in the treatment of ALS of a pre-symptomatic human subject whose onset of clinical manifestations of ALS has been predicted by the method of any one of claims 1-8.

10. The ALS therapy of claim 9, wherein the ALS therapy is selected from the group consisting of edaravone, riluzole, mesenchymal stem cells, copper-ATSM (CuATSM), AB-1010 (masitinib), CK-2017357(tirasemtiv), E0302 (mecobalamin), NP001, pyrimethamine, ibudilast, glial restricted progenitor cells, mexiletine, memantine, GDC-0134, EPI-589, RNS-60, pimozide, tocilizumab, ezogabine, ODM-109, low-dose IL-2, amylyx, an antisense oligonucleotide or viral vector-mediated gene therapy, and combinations thereof.

11. The ALS therapy of claim 10, wherein the antisense oligonucleotide or viral vector-mediated gene therapy targets one or more genetic mutation(s) associated with ALS.

12. The ALS therapy of claim 11, wherein the antisense oligonucleotide is ISIS-SOD1Rx (BIIB067), C9orf72, ASO-816, ASO061, ISIS SMNRx or ISIS 333611 or combinations thereof.

13. The ALS therapy of claim 11, wherein the viral vector-mediated gene therapy is VY-SOD101.

14. The ALS therapy of any one of claims 9-13, wherein use of an ALS therapy is performed within 12 months of predicting the onset of ALS.

## Patentansprüche

1. Verfahren zum Vorhersagen des Auftretens klinischer Manifestationen von amyotropher Lateralsklerose (ALS) in einem präsymptomatischen menschlichen Individuum, wobei das Verfahren umfasst
(a) Messen der Werte der phosphorylierten schweren Kette von Neurofilament (pNfH) und/oder der leichten Kette von Neurofilament (NfL) in einer Probe eines Individuums in einer präsymptomatischen Phase, und
(b) Vorhersagen des Auftretens von ALS,
wobei erhöhte Werte von pNfH und/oder NfL relativ zu den Werten von pNfH und/oder NfL, die früher bei dem Individuum gemessen wurden, das Auftreten klinischer Manifestationen von ALS signalisieren.

2. Verfahren nach Anspruch 1, wobei Schritt (a) umfasst
(a1) Messen der pNfH- und/oder NfL-Werte in einer Probe des Individuums zu einem ersten Zeitpunkt, und
(a2) Messen der pNfH- und/oder NfL-Werte in einer Probe des Individuums zu einem zweiten Zeitpunkt, und
(a3) Vergleichen der Werte von pNfH und/oder NfL von (a1) und (a2).

3. Verfahren nach Anspruch 2, wobei zwischen dem ersten Zeitpunkt und dem zweiten Zeitpunkt drei bis sechs Monate liegen.

4. Verfahren nach Anspruch 1, wobei Schritt (b) das Nachweisen von NfL-Werten von mindestens 24 pg/ml umfasst.

5. Verfahren nach einem der Ansprüche 1 bis 4, wobei das Individuum ein präsymptomatischer menschlicher Träger einer mit ALS assoziierten Genmutation ist.

6. Verfahren nach Anspruch 5, wobei der präsymptomatische menschliche Träger einer mit ALS assoziierten Genmutation eine Mutation in Superoxiddismutase 1 (SOD1), Chromosom-9-offener-Leserahmen-72 (C9orf72), Fused in Sarcoma (FUS), TAR-DNA-Bindungsprotein (TARDBP), Valosin-haltiges Protein (VCP), Angiogenin (ANG), Coiled-Coil-Helix-Coiled-Coil-Helix-Domäne enthaltend 10 (CHCHD10), chromatinmodifizierendes Protein 2B (CHMP2B), heterogenes nukleäres Ribonukleoprotein A1 (HNRNPA1), Matrin 3 (MATR3), Optineurin (OPTN), Profilin 1 (PFN1), Spatacsin (SPG11), Sequestosom 1 (SQSTM1), TDP-43 (TARDP), TANKbindende Kinase 1 (TBK1), Tubulin Alpha 1 (TUBA4A), Ubiquilin-2 (UBQLN2) oder eine Kombination davon umfasst.

7. Verfahren nach einem der Ansprüche 1 bis 6, wobei Schritt (a) das Messen der Werte von neurofilamenthaltigen Hetero-Aggregaten oder Spaltungsprodukte von pNfH und/oder NfL umfasst.

8. Verfahren nach einem der Ansprüche 1 bis 7, wobei die pNfH- und NfL-Proteinwerte durch antikörperbasierte Immunassays gemessen werden.

9. ALS-Therapie zur Verwendung bei der Behandlung von ALS bei einem präsymptomatischen menschlichen Individuum, dessen Auftreten klinischer Manifestationen von ALS durch das Verfahren nach einem der Ansprüche 1 bis 8 vorhergesagt wurde.

10. ALS-Therapie nach Anspruch 9, wobei die ALS-Therapie ausgewählt ist aus der Gruppe bestehend aus Edaravon, Riluzol, mesenchymalen Stammzellen, Kupfer-ATSM (CuATSM), AB-1010 (Masitinib), CK-2017357 (Tirasemtiv), E0302 (Mecobalamin), NP001, Pyrimethamin, Ibudilast, auf Glia beschränkte Vorläuferzellen, Mexiletin, Memantin, GDC-0134, EPI-589, RNS-60, Pimozid, Tocilizumab, Ezogabin, ODM-109, niedrig dosiertes IL-2, Amylyx, ein Antisense-Oligonukleotid oder eine Virusvektor-vermittelte Gentherapie und Kombinationen davon.

11. ALS-Therapie nach Anspruch 10, wobei das Antisense-Oligonukleotid oder die Virusvektor-vermittelte Gentherapie auf eine oder mehrere Genmutation(en) im Zusammenhang mit ALS abzielt.

12. ALS-Therapie nach Anspruch 11, wobei das Antisense-Oligonukleotid ISIS-SOD1Rx (BIIB067), C9orf72, ASO-816, ASO061, ISIS SMNRx oder ISIS 333611 oder Kombinationen davon ist.

13. ALS-Therapie nach Anspruch 11, wobei die Virusvektor-vermittelte Gentherapie VY-SOD101 ist.

14. ALS-Therapie nach einem der Ansprüche 9 bis 13, wobei die Anwendung einer ALS-Therapie innerhalb von 12 Monaten nach der Vorhersage des Auftretens von ALS durchgeführt wird.

## Revendications

1. Procédé de prédiction de l'apparition de manifestations cliniques de la sclérose latérale amyotrophique (SLA) chez un sujet humain présymptomatique, le procédé comprenant
(a) la mesure des taux de chaîne lourde des neurofilaments phosphorylés (pNfH) et/ou de chaîne légère des neurofilaments (NfL) dans un échantillon d'un sujet en phase présymptomatique, et
(b) la prédiction de l'apparition de la SLA,
dans lequel des taux élevés de pNfH et/ou de NfL par rapport aux taux de pNfH et/ou de NfL mesurés chez le sujet plus tôt dans le temps signalent l'apparition de manifestations cliniques de la SLA.

2. Procédé selon la revendication 1, dans lequel l'étape (a) comprend
(a1) la mesure des taux de pNfH et/ou de NfL dans un échantillon du sujet à un premier point dans le temps, et
(a2) la mesure des taux de pNfH et/ou de NfL dans un échantillon du sujet à un second point dans le temps, et
(a3) la comparaison des taux de pNfH et/ou de NfL de (a1) et (a2).

3. Procédé selon la revendication 2, dans lequel le premier point dans le temps et le second point dans le temps sont espacés d'environ trois à six mois.

4. Procédé selon la revendication 1, dans lequel l'étape (b) comprend la détection de taux de NfL d'au moins 24 pg/ml.

5. Procédé selon l'une quelconque des revendications 1 à 4, dans lequel le sujet est un porteur humain présymptomatique d'une mutation génétique associée à la SLA.

6. Procédé selon la revendication 5, dans lequel le porteur humain présymptomatique d'une mutation génétique associée à la SLA comprend une mutation dans la superoxyde dismutase 1 (SOD1), le cadre de lecture ouvert 72 sur le chromosome 9 (C9orf72), fusionné dans le sacorme (FUS), la protéine de liaison TAR-ADN (TARDBP), la protéine contenant de la valosine (VCP), l'angiogénine (ANG), le domaine coiled-coil-hélice-coiled-coil-hélice contenant 10 (CHCHD10), la protéine de modification de la chromatine 2B (CHMP2B), la ribonucléoprotéine nucléaire hétérogène A1 (HNRNPA1), la matrine 3 (MATR3), l'optineurine (OPTN), la profiline 1 (PFN1), la spatacine (SPG11), la séquestosome 1 (SQSTM1), TDP-43 (TARDP), la kinase se liant à TANK 1 (TBK1), la tubuline alpha 1 (TUBA4A), l'ubiquiline-2 (UBQLN2), ou une combinaison de ceux-ci.

7. Procédé selon l'une quelconque des revendications 1 à 6, dans lequel l'étape (a) comprend la mesure des taux d'hétéro-agrégats contenant des neurofilaments, ou de produits de clivage de pNfH et/ou NfL.

8. Procédé selon l'une quelconque des revendications 1 à 7, dans lequel les taux de protéines pNfH et NfL sont mesurés par des immunoessais basés sur des anticorps.

9. Thérapie contre la SLA pour une utilisation dans le traitement de la SLA d'un sujet humain présymptomatique dont l'apparition des manifestations cliniques de la SLA a été prédite par le procédé selon l'une quelconque des revendications 1 à 8.

10. Thérapie contre la SLA selon la revendication 9, dans laquelle la thérapie contre la SLA est choisie parmi le groupe constitué par l'édaravone, le riluzole, les cellules souches mésenchymateuses, le cuivre-ATSM (CuATSM), AB-1010 (masitinib), CK-2017357 (tirasemtiv), E0302 (mécobalamine), NP001, la pyriméthamine, l'ibudilast, les cellules progénitrices gliales restreintes, la mexilétine, la mémantine, GDC-0134, EPI-589, RNS-60, le pimozide, le tocilizumab, l'ézogabine, ODM-109, l'IL-2 à faible dose, l'amylyx, un oligonucléotide antisens ou une thérapie génique médiée par un vecteur viral, et des combinaisons de ceux-ci.

11. Thérapie contre la SLA selon la revendication 10, dans laquelle l'oligonucléotide antisens ou la thérapie génique médiée par un vecteur viral cible une ou plusieurs mutations génétiques associées à la SLA.

12. Thérapie contre la SLA selon la revendication 11, dans laquelle l'oligonucléotide antisens est ISIS-SOD1Rx (BIIB067), C9orf72, ASO-816, ASO061, ISIS SMNRx ou ISIS 333611 ou des combinaisons de ceux-ci.

13. Thérapie contre la SLA selon la revendication 11, dans laquelle la thérapie génique médiée par un vecteur viral est VY-SOD101.

14. Thérapie contre la SLA selon l'une quelconque des revendications 9 à 13, dans laquelle l'utilisation d'une thérapie contre la SLA est réalisée dans les 12 mois qui suivent la prédiction de l'apparition de la SLA.
